# EUROPÄISCHE PATENTSCHRIFT

(11) **EP 0 783 277 B1**
(45) Veröffentlichungstag und Bekanntmachung des Hinweises auf die Patenterteilung: **10.07.2002**
(21) Anmeldenummer: 95917880.7
(22) Anmeldetag: 22.05.1995
(51) Int. Cl.: A61B 17/70

(54) **KLEMMBACKE FÜR EINE WIRBELSÄULEN-FIXATIONSVORRICHTUNG**
CLAMP JAW FOR A SPINAL FIXATION DEVICE
MACHOIRE DE SERRAGE D'UN DISPOSITIF DE CONTENTION POUR LA COLONNE VERTEBRALE

(43) Veröffentlichungstag der Anmeldung: 16.07.1997
(73) Patentinhaber: Synthes AG, Chur, 7002 Chur (CH)
(72) Erfinder: AMREIN, Thomas, CH-6048 Horw (CH); TAGWERKER, Konrad, CH-4053 Basel (CH); JEANNERET, Bernhard, CH-9402 Mörschwil (CH)
(74) Vertreter: Lusuardi, Werther Giovanni, Dr.
(86) Internationale Anmeldenummer: CH9500114
(87) Internationale Veröffentlichungsnummer: WO9637159

(56) Entgegenhaltungen:
- EP-A- 0 441 668
- EP-A- 0 468 264
- WO-A-93/14721
- US-A- 5 403 314

## Beschreibung

Die Erfindung bezieht sich auf eine Klemmbacke für eine Wirbelsäulen-Fixationsvorrichtung gemäss dem Oberbegriff des Patentanspruchs 1.

Eine solche Klemmbacke ist beispielsweise aus der EP-A-0 553 424 bekannt. Sie dient dazu die einzelnen Wirbelkörper in einer definierbaren gegenseitigen Lage zueinander zu fixieren, wozu eine Anzahl in die Wirbelkörper implantierter Knochenschrauben mittels solcher Klemmbacken an einem in Richtung der Wirbelsäule verlaufenden Längsträger fixiert werden.

Die bekannte Klemmbacke gemäss der EP-A-0 553 424 ist dreiteilig ausgeführt um sowohl den Abstand als auch den Winkel zwischen Knochenschraube und Längsträger einstellen zu können. Gerade diese grosse Flexibilität erschwert die Operationstechnik in erheblichem Masse. Winkel und Abstand zwischen der Knochenschraube und dem Längsträger müssen in situ eingestellt werden. Hierzu muss die Klemmbacke und der Querträger gleichzeitig ausgerichtet werden, und mit der Fixierschraube die gewünschte Stellung des Konstrukts fixiert werden. Dabei muss mit einem Gabelschlüssel über den Sechskant ein Gegenmoment zum Anziehmoment der Fixierschraube aufgebracht werden, damit sich das ganze Konstrukt beim Festziehen der Fixierschraube nicht verdreht. In den engen Verhältnissen an der Halswirbelsäule ist die Montage mehrerer solcher Konstrukte äusserst mühsam, da die Sicht auf die zu montierenden Teile begrenzt ist, und sich die Operateure gegenseitig beim Ausrichten und Fixieren der einzelnen Komponenten behindern.

Aus der US-A-5403314 CURRIER ist eine weitere Klemmbacke für eine Wirbelsäulen-Fixationsvorrichtung gemäss dem Oberbegriff des Anspruchs 1 bekannt. Diese bekannte Vorrichtung besitzt einen relativ voluminösen keilförmigen Fortsatz mit einem durchgehenden Schraubenloch zur Aufnahme einer Knochenschraube, mit der die Klemmback am Knochen befestigt werden kann. Der voluminöse Fortsatz hat den Nachteil, dass er einerseits invasiv ist und anderseits die Verwendung einer stärkeren Röntgenstrahlendosis bedingt, sei dies intra- oder postoperativ bei der Kontrolle des Implantates.

Hier will die Erfindung Abhilfe schaffen. Der Erfindung liegt die Aufgabe zugrunde, eine Klemmbacke zu schaffen, welche einfach und kostengünstig herstellbar ist und das intraoperative Einstellen der Abstände und Winkel zwischen den von der Klemmbacke gehaltenen Strukturen überflüssig macht und damit den operativen Zeitaufwand reduziert und die Sicherheit erhöht.

Die Erfindung löst die gestellte Aufgabe mit einer Klemmbacke, welche die Merkmale des Anspruchs 1 aufweist. Weitere vorteilhafte Ausgestaltungen der Erfindung sind in den abhängigen Ansprüchen gekennzeichnet.

Die durch die Erfindung erreichten Vorteile sind im wesentlichen darin zu sehen, dass dank der erfindungsgemässen Klemmbacke die anatomisch richtige Lage der Knochenschraube zum Längsträger durch deren Geometrie vorgegeben ist. Sie braucht daher nicht mehr - wie beim Stand der Technik - in situ eingestellt zu werden, was Zeit spart und die Sicherheit einer anatomisch richtigen Verankerung im Knochen erhöht. In situ müssen nur noch die Knochenschrauben durch die Klemmbacken in den Knochen eingedreht werden.
Ein weiterer Vorteil gegenüber dem Stand der Technik besteht darin, dass die Gefahr sich lösender Verbindungen innerhalb der Klemmbacke nicht existiert, da die erfindungsgemässe Klemmbacke aus einem einzigen Teil besteht.

Die Erfindung und Weiterbildungen der Erfindung werden im folgenden anhand der teilweise schematischen Darstellungen eines Ausführungsbeispiels noch näher erläutert.

Es zeigen:
Fig. 1 eine perspektivische Darstellung der erfindungsgemässen Klemmbacke;
Fig. 2 einen schematischen Grundriss der Klemmbacke nach Fig. 1;
Fig. 3 einen schematischen Seitenriss der Klemmbacke nach Fig. 1 ;
Fig. 4 perspektivische Darstellung der erfindungsgemässen Klemmbacke mit eingesetztem Längsträger und Knochenschraube; und
Fig. 5 eine perspektivische Darstellung der erfindungsgemässen Klemmbacke nach Fig. 4, wobei die Knochenschraube in einem Wirbelknochen verankert ist.

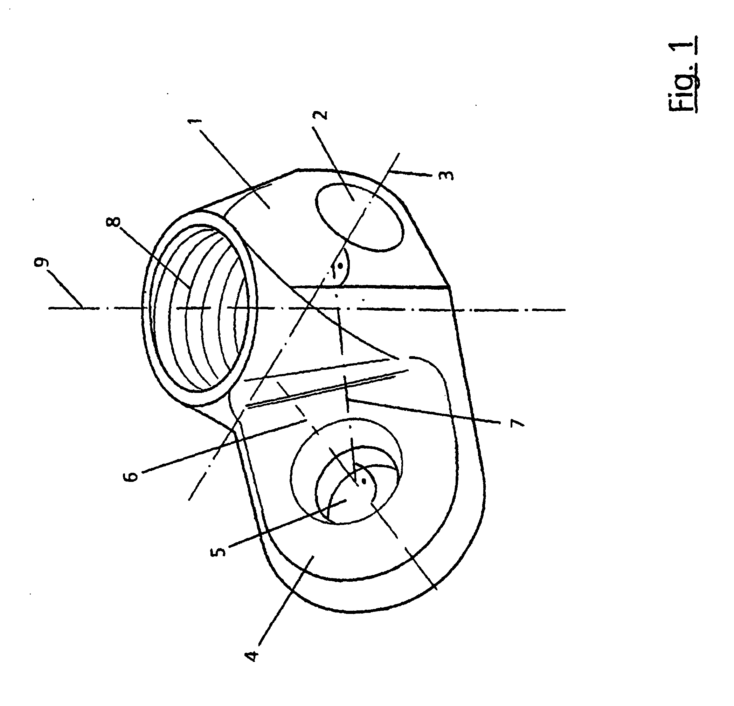

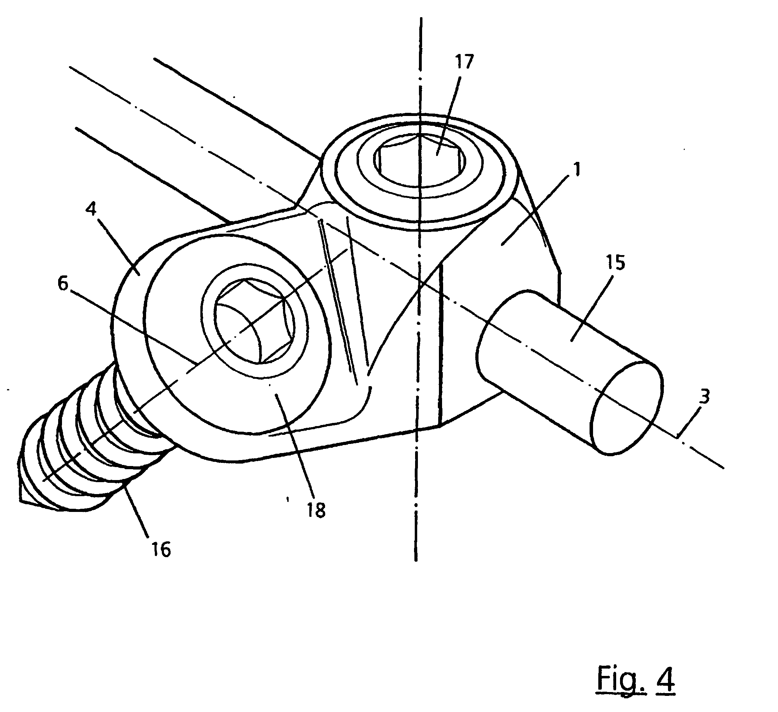

Die in Fig. 1 dargestellte Klemmbacke für eine Wirbelsäulen-Fixationsvorrichtung umfasst im wesentlichen einen Grundkörper 1 mit einem durchgehenden Kanal 2 mit der Längsachse 3 zur Aufnahme eines Längsträgers 15 (Fig. 4) sowie einen, sich am Grundkörper 1 anschliessenden, im wesentlichen planaren Fortsatz 4, der ein durchgehendes Schraubenloch 5 mit der Mittelachse 6 zur Aufnahme einer Knochenschraube 16 (Fig. 4) aufweist. Die Klemmbacke ist somit - abgesehen von den darin einzuführenden Elementen 15,16 - einteilig ausgebildet.

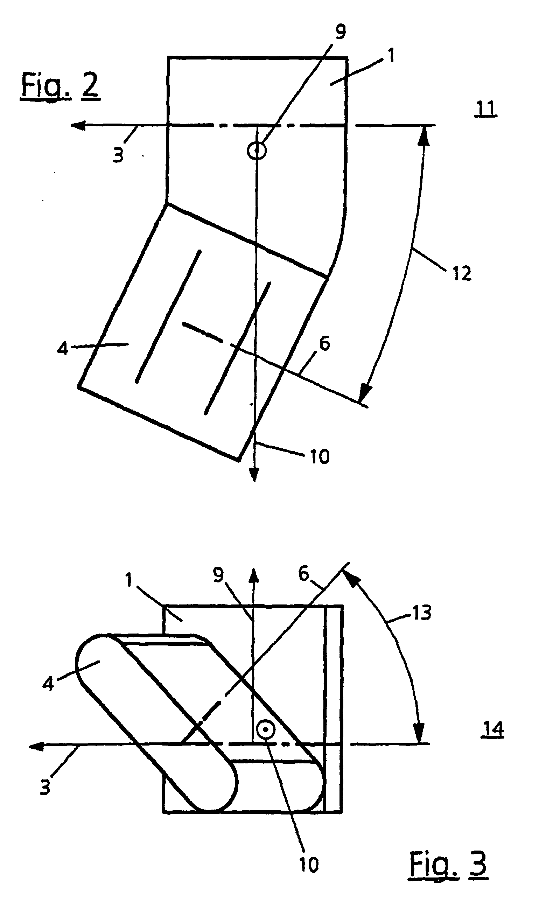

Zum besseren Verständnis der in den Fig. 2 und 3 dargestellten Projektionen der Klemmbacke ist in Fig. 1 die sowohl auf der Längsachse 3 als auf der Längsachse 9 senkrecht stehende Orthogonale 10 dargestellt, welche in anatomischer Hinsicht der medio-lateralen Richtung entspricht.

In Fig. 2 ist eine Projektion der Klemmbacke in die durch Längsachse 3 und Orthogonale 10 gebildeten Ebene 11 dargestellt. Die Längsachse 9 steht somit senkrecht auf der Ebene 11, welche in Fig. 2 der Zeichenebene entspricht. In anatomischer Hinsicht entspricht die Ebene 11 der Medianebene.

Der Kanal 2 ist mit Mitteln versehen, um einen in den Kanal 2 eingeführten Längsträger 15 longitudinal und rotativ blockieren zu können. Diese Mittel bestehen vorzugsweise aus einer im Grundkörper 1 angebrachten Gewindebohrung 8 mit zur Ebene 11 orthogonal stehender Längsachse 9, in welche eine Stellschraube 17 (Fig. 4) einführbar ist. Die Längsachse 9 entspricht in anatomischer Hinsicht der dorsalen Richtung.

Die Längsachse 3 welche in anatomischer Hinsicht der kranialen Richtung entspricht und die Mittelachse 6 bilden windschiefe Geraden, deren Abstand 7 unveränderlich ist und im Bereich von 4 - 10 mm liegt. Vorzugsweise liegt der Abstand 7 zwischen 5 - 8 mm und typischerweise zwischen 5,8 - 6,7 mm.

Der Kanal 2 weist eine in axialer Richtung gemessene Länge von 5 - 7 mm, vorzugsweise von 5,7 - 6,3 mm auf. Sein Durchmesser beträgt 3,0 bis 4,5 mm, vorzugsweise 3,2 bis 3,8 mm (z.B. 3,5 mm). Der Kanal 2 ist in der zeichnerisch dargestellten Ausführung geschlossen ausgeführt; er kann jedoch auch eine seitliche Öffnung aufweisen (auf der dem Fortsatz 4 entgegengesetzten Seite des Grundkörpers 1), damit ein Längsträger 15 auch seitlich durch diese Längsöffnung des Kanals 2 eingelegt werden kann. Diese Ausführungsform des Kanals 2 erlaubt auch noch intraoperativ einen Umbau der Wirbelsäulen-Fixationsvorrichtung ohne dass die Klemmbacke vollständig demontiert werden muss, bzw. der Längsträger 15 in axialer Richtung ganz aus dem Kanal 2 herausgezogen werden muss.

Das Schraubenloch 5 ist im Aufnahmebereich des Kopfes einer darin einzuführenden Knochenschraube konkav, vorzugsweise sphärisch ausgebildet, um eine gewisse Angulation der mit einem mit Kugelkopf 18 versehenen Knochenschraube 16 (Fig. 4) zu gestatten.

Der in diese Ebene 11 projizierte laterale Winkel 12 zwischen Längsachse 6 und Längsachse 3 liegt für Klemmbacken für die Wirbel C3-C7 im Bereich von 20° bis 30°. Im gezeichneten Beispiel beträgt der laterale Winkel 12 exakt 25°.

In Fig. 3 ist eine Projektion der Klemmbacke in die durch Längsachse 3 und senkrecht zur Orthogonalen 10 stehenden Ebene 14 dargestellt. Die Ebene 14 entspricht in Fig. 3 der Zeichenebene. In anatomischer Hinsicht entspricht die Ebene 14 der Sagittalebene.
Der in diese Ebene 14 projizierte kraniale Winkel 13 zwischen Längsachse 6 und Längsachse 3 liegt bei Klemmbacken für die Wirbel C3-C7 sowie C2 vorzugsweise im Bereich von 42,5° bis 50,0°. Im gezeichneten Beispiel beträgt der kraniale Winkel 14 exakt 47°49'.
Bei Klemmbacken für die Wirbel T1-T2 beträgt der kraniale Winkel 14 vorzugsweise 80° - 100°.

Die in den Fig. 1 - 3 dargestellte Klemmbacke entspricht einer linksseitigen Ausführung. Die entsprechende rechtsseitige Ausführung erhält man durch Spiegelung der Klemmbacke an der Ebene 14 (Sagittalebene), wobei alle geometrischen Beziehungen (Abstände und Winkel) gleich bleiben.

In Fig. 4 ist die Klemmbacke mit eingeführtem und mittels der Stellschraube 17 blockiertem Längsträger 15 sowie mit einer durch den Fortsatz 4 eingeführten Knochenschraube 16 mit Kugelkopf 18 dargestellt.

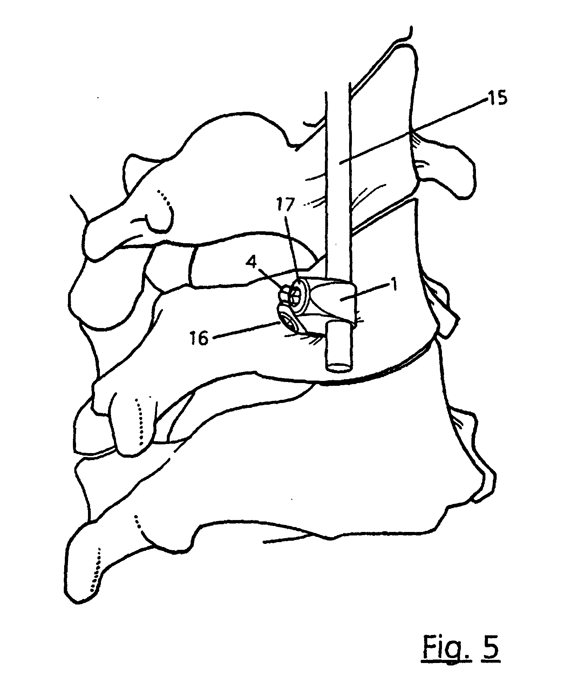

In Fig. 5 ist die gleiche Klemmbacke nach Fig. 4 dargestellt, welche nun mittels der durch den Fortsatz 4 eingeführten Knochenschraube 16 im Wirbelknochen verankert ist. Über den mittels der Stellschraube 17 blockierten Längsträger 15 lässt sich die Verbindung zu einer oder mehreren weiteren Klemmbacken herstellen, welche mittels weiterer Knochenschrauben an anderen Wirbelknochen verankert sind.

Der Fortsatz 4 ist in der in den Fig. 1 - 5 dargestellten Ausführungsform der Klemmbacke im wesentlichen tafelförmig ausgebildet, wobei die Tafelebene in einem Winkel von etwa 45° zur Projektionsebene 11 angeordnet ist. Der Fortsatz 4 kann aber auch als Keil ausgebildet sein, wobei vorzugsweise die eine Keilfläche parallel zur Projektionsebene 11 und die andere Keilfläche in einem Winkel von etwa 45° zur Projektionsebene 11 angeordnet ist. Diese Ausführungsform hat den Vorteil, dass sich die Klemmbacke mit der parallel zur Projektionsebene 11 verlaufenden Keilfläche am Wirbelknochen abstützen kann.

Im folgenden wird noch ein spezifisches Beispiel für eine erfindungsgemässe Klemmbacken angegeben, mit welchem besonders gute Ergebnisse erzielt werden konnte:

### Beispiel

| | |
|---|---|
| Länge des Kanal 2 | 6 mm |
| kranialer Winkel 13 | 47,5° |
| lateraler Winkel 12 | 25° |
| Abstand 7 | 6,30 mm |

Die erfindungsgemässe Klemmbacke gestattet das Setzen von Knochenschrauben in einem genau definierten Winkel zur Anatomie im Wirbelsäulenbereich (kranialer und lateraler Winkel) , was zu einer sicheren und stabilen Verankerung im Knochen führt. Beispielsweise können die von MAGERL vorgeschlagenen Winkel eingehalten werden, ohne dazu aufwendige Zielvorgänge durchzuführen.
Die Dimensionierung der erfindungsgemässen Klemmbacke gestattet ein gutes Anlagern von Knochenspan, so dass eine gute Fusion erreicht wird. Es sind auch Korrekturen, wie Kompression und Distraktion möglich.

## Patentansprüche

1. Klemmbacke für eine Wirbelsäulen-Fixationsvorrichtung mit
A) einem Grundkörper (1), der einen durchgehenden Kanal (2) mit der Längsachse (3) zur Aufnahme eines Längsträgers (15) aufweist;
B) der Grundkörper (2) mit Mitteln versehen ist, um einen in den Kanal (2) eingeführten Längsträger longitudinal und rotativ blockieren zu können, wobei die Mittel eine Zentralachse (9) aufweisen;
C) einem, sich am Grundkörper (1) anschliessenden Fortsatz (4), der ein durchgehendes Schraubenloch (5) mit der Mittelachse (6) zur Aufnahme einer Knochenschraube (16) aufweist; wobei
D) die Längsachse (3) und die Mittelachse (6) windschiefe Geraden sind;
E) der Abstand (7) zwischen Längsachse (3) und Mittelachse (6) unveränderlich ist;
F) der Fortsatz (4) in einem Winkel von 0° bis 45° zu einer ersten Projektionsebene (11) angeordnet ist; wobei
G) die erste Projektionsebene (11) von der Längsachse (3) und einer auf letzerer und der Zentralachse (9) senkrecht stehenden Orthogonalen (10) aufgespannt ist;
**dadurch gekennzeichnet, dass**
H) der Abstand (7) zwischen Längsachse (3) und Mittelachse im Bereich von 4 - 10 mm liegt; und
I) der laterale Winkel (12) zwischen Mittelachse (6) und Längsachse (3) in der ersten Projektionsebene (11) im Bereich von +20° bis +30° liegt.

2. Klemmbacke nach Anspruch 1, **dadurch gekennzeichnet, dass** der Abstand (7) im Bereich von 5 - 8 mm, vorzugsweise von 5,8 - 6,7 mm liegt.

3. Klemmbacke nach Anspruch 1 oder 2, **dadurch gekennzeichnet, dass** der Kanal (2) eine in axiale Richtung gemessene Länge von 5 - 7 mm, vorzugsweise von 5,7 - 6,3 mm aufweist.

4. Klemmbacke nach einem der Ansprüche 1 - 3, **dadurch gekennzeichnet, dass** der Kanal (2) einen Durchmesser im Bereich von 3,0 bis 4,5 mm, vorzugsweise von 3,2 bis 3,8 mm aufweist.

5. Klemmbacke nach einem der Ansprüche 1 - 4, **dadurch gekennzeichnet, dass** die Mittel zur Blockierung eines in den Kanal (2) eingeführten Längsträgers eine im Grundkörper (1) angebrachte Gewindebohrung (8) mit zur ersten Projektionsebene (11) orthogonal stehender Längsachse (9) umfassen, in welche eine Stellschraube (17) einführbar ist.

6. Klemmbacke nach einem der Ansprüche 1 - 5, **dadurch gekennzeichnet, dass** das Schraubenloch (5) im Aufnahmebereich des Kopfes einer darin einzuführenden Knochenschraube konkav, vorzugsweise sphärisch ausgebildet ist.

7. Klemmbacke nach einem der Ansprüche 1 - 6, **dadurch gekennzeichnet, dass** sie einteilig ausgeführt ist.

8. Klemmbacke nach einem der Ansprüche 1 - 7, **dadurch gekennzeichnet, dass** der Fortsatz (4) tafelförmig ausgebildet ist.

9. Klemmbacke nach einem der Ansprüche 1 - 7, **dadurch gekennzeichnet, dass** der Fortsatz (4) keilförmig ausgebildet ist.

10. Klemmbacke nach einem der Ansprüche 1 - 9, **dadurch gekennzeichnet, dass** der kraniale Winkel (13) zwischen Mittelachse (6) und Längsachse (3) in einer durch Längsachse (3) und Zentralachse (9) gebildeten zweiten Projektionsebene (14) im Bereich von 42,5° bis 50° liegt.

11. Klemmbacke nach einem der Ansprüche 1 - 9, **dadurch gekennzeichnet, dass** der kraniale Winkel (13) zwischen Mittelachse (6) und Längsachse (3) in einer durch Längsachse (3) und Zentralachse (9) gebildeten zweiten Projektionsebene (14) im Bereich von 80° bis 100° liegt.

12. Klemmbacke nach einem der Ansprüche 1 - 11, **dadurch gekennzeichnet, dass** der Fortsatz (4) als Keil ausgebildet ist, wobei die eine Keilfläche parallel zur ersten Projektionsebene (11) und die andere Keilfläche in einem Winkel von 0° bis 45° zur zweiten Projektionsebene (11) angeordnet ist.

13. Klemmbacke nach einem der Ansprüche 1 - 11, **dadurch gekennzeichnet, dass** der Kanal (2) auf der dem Fortsatz (4) entgegengesetzten Seite offen ausgebildet ist zur seitlichen Aufnahme eines Längsträgers (15).

14. Klemmbacke nach einem der Ansprüche 1 - 13, **dadurch gekennzeichnet, dass** Ober- und Unterseite des tafelförmigen Fortsatzes (4) im wesentlichen parallel zueinander verlaufen.

## Claims

1. Clamp for a spinal affixation device comprising
A) a base body (1) crossed by a duct (2) for receiving a longitudinal support (15), the duct (2) having a longitudinal axis (3);
B) the base body (1) having means to rotationally and longitudinally lock a longitudinal support being inserted into the duct (2), the means having a central axis (9);
C) an extension (4) adjacent the base body (1) and crossed by a screw hole (5) for receiving a bone screw (16) and having a center axis (6); whereby
D) the longitudinal axis (3) and the center axis (6) are skewed straight lines;
E) the distance (7) between the longitudinal axis (3) and the center axis (6) being invariable;
F) the extension (4) being mounted at an angle of between 0° and 45° with respect to a first plane of projection (11); whereby
G) the first plane of projection (11) is formed by the longitudinal axis (3) and an orthogonal line (10) perpendicular the latter and the central axis (9)
**characterized in that**
H) the spacing (7) between the longitudinal axis (3) and the center axis (6) is in the range between 4 - 10 mm; and
I) the azimuth angle (12) subtended by the center axis (6) and the longitudinal axis (3) in the first plane of projection (11) is in the range of +20° and +30°.

2. Clamp according to claim 1, wherein the spacing (7) is in the range of 5 to 8 mm, preferably of 5,8 to 6,7mm.

3. Clamp according to claim 1 or 2, wherein the length of the duct (2) along the longitudinal axis (3) is 5 to 7 mm, preferably 5,7 to 6,3 mm.

4. Clamp according to one of the claims 1 to 3, wherein the duct (2) has a diameter in the range of 3,0 to 4,5 mm, preferably of 3,2 to 3,8 mm.

5. Clamp according to one of the claims 1 to 4, wherein the means to lock a longitudinal support being inserted into the duct (2) includes a threaded borehole (8) located within the base body (1) and having a central axis (9) orthogonal to the first plane of projection (11) and wherein an adjusting screw (17) is insertable.

6. Clamp according to one of the claims 1 to 5, wherein the screw hole (5) is configured concave, preferably spherical in the range of the acceptance of the head of a bone screw which is insertable therethrough.

7. Clamp according to one of the claims 1 to 6, wherein it is formed from a single piece.

8. Clamp according to one of the claims 1 to 7, wherein the extension (7) is tabularly configured.

9. Clamp according to one of the claims 1 to 7, wherein the extension (7) is configured wedge-like.

10. Clamp according to one of the claims 1 to 9, wherein the cranial angle (13) between the center axis (6) and the longitudinal axis (3) in a second plane of projection (14) formed by the longitudinal axis (3) and the central axis (9) is in the range of 42,5° to 50°.

11. Clamp according to one of the claims 1 to 9, wherein the cranial angle (13) between the center axis (6) and the longitudinal axis (3) in a second plane of projection (14) formed by the longitudinal axis (3) and the central axis (9) is in the range of 80° to 100°.

12. Clamp according to one of the claims 1 to 11, wherein the extension (4) is configured as a wedge, whereby one wedge surface is parallel to the first plane of projection (11) and the other wedge surface is at an angle of 0° to 45° to the second plane of projection (14).

13. Clamp according to one of the claims 1 to 11, wherein the duct (2) includes an aperture on the side opposite the extension (4) for receiving a longitudinal support (15).

14. Clamp according to one of the claims 1 to 13, wherein the upper and lower surfaces of the tabular extension (4) are essentially parallel to each other.

## Revendications

1. Mâchoire de serrage pour un dispositif de contention dans la colonne vertébrale, comportant :
A) un corps de base (1) qui est traversé par un canal (2) présentant un axe longitudinal (3) pour la réception d'un support longitudinal (15) ;
B) le corps de base (2) étant doté de moyens pour pouvoir bloquer longitudinalement et en rotation un support longitudinal inséré dans le canal (2), les moyens présentant un axe central (9) ;
C) un appendice (4) qui se raccorde au corps de base (1) et qui est traversé par un trou fileté (5) présentant un axe central (6), pour la réception d'une vis à os (16) ;
D) l'axe longitudinal (3) et l'axe central (6) étant des droites gauches ;
E) la distance (7) entre l'axe longitudinal (3) et l'axe central 6) étant invariable ;
F) l'appendice (4) étant disposé sous un angle de 0° à 45° par rapport à un premier plan de projection (11) ; et
G) le premier plan de projection (11) étant formé par l'axe longitudinal (3) et une normale (10) perpendiculaire à ce dernier et à l'axe central (9) ;
**caractérisée en ce que**
H) la distance (7) entre l'axe longitudinal (3) et l'axe central est située dans la plage de 4 à 10 mm ; et
I) l'angle latéral (12) entre l'axe central (6) et l'axe longitudinal (3) dans le premier plan de projection (11) est situé dans la plage de +20° à +30°.

2. Mâchoire de serrage selon la revendication 1, **caractérisée en ce que** la distance (7) est située dans la plage de 5 à 8 mm, de préférence de 5,8 à 6,7 mm.

3. Mâchoire de serrage selon la revendication 1 ou 2, **caractérisée en ce que** le canal (2) présente dans la direction axiale une longueur de 5 à 7 mm, de préférence de 5,7 à 6,3 mm.

4. Mâchoire de serrage selon l'une des revendications 1 à 3, **caractérisée en ce que** le canal (2) présente un diamètre dans la plage de 3,0 à 4,5 mm, de préférence de 3,2 à 3,8 mm.

5. Mâchoire de serrage selon l'une des revendications 1 à 4, **caractérisée en ce que** les moyens de blocage d'un support longitudinal inséré dans le canal (2) comprennent un alésage fileté (8) ménagé dans le corps de base (1) et dont l'axe longitudinal (9) est perpendiculaire au premier plan de projection (11), et dans lequel une vis de réglage (17) peut être insérée.

6. Mâchoire de serrage selon l'une des revendications 1 à 5, **caractérisée en ce que** dans la région de réception de la tête d'une vis à os qui doit y être insérée, le trou fileté (5) présente une configuration concave et de préférence sphérique.

7. Mâchoire de serrage selon l'une des revendications 1 à 6, **caractérisée en ce qu'**elle est réalisée d'une seule pièce.

8. Mâchoire de serrage selon l'une des revendications 1 à 7, **caractérisée en ce que** l'appendice (4) est configuré en forme de table.

9. Mâchoire de serrage selon l'une des revendications 1 à 7, **caractérisée en ce que** l'appendice (4) est configuré en forme de biseau.

10. Mâchoire de serrage selon l'une des revendications 1 à 9, **caractérisée en ce que** l'angle crânial (13) entre l'axe central (6) et l'axe longitudinal (3) dans un deuxième plan de projection formé par l'axe longitudinal (3) et l'axe central (9) est compris dans la plage de 42,5° à 50°.

11. Mâchoire de serrage selon l'une des revendications 1 à 9, **caractérisée en ce que** l'angle crânial (13) entre l'axe central (6) et l'axe longitudinal (3) dans un deuxième plan de projection (14) formé par l'axe longitudinal (3) et l'axe central (9) est compris dans la plage de 80° à 100°.

12. Mâchoire de serrage selon l'une des revendications 1 à 11, **caractérisée en ce que** l'appendice (4) est configuré comme biseau, l'une des surfaces du biseau étant disposée parallèlement au premier plan de projection (11) et l'autre surface du biseau étant disposée à un angle de 0° à 45° par rapport au deuxième plan de projection (11).

13. Mâchoire de serrage selon l'une des revendications 1 à 11, **caractérisée en ce que** le canal (2) est ouvert sur le côté opposé à l'appendice (4), pour la réception latérale d'un support longitudinal (15).

14. Mâchoire de serrage selon l'une des revendications 1 à 13, **caractérisée en ce que** le côté supérieur et le côté inférieur de l'appendice (4) en forme de table s'étendent essentiellement en parallèle l'un à l'autre.
